# EUROPÄISCHE PATENTANMELDUNG

(11) **EP 2 230 026 A1**
(43) Veröffentlichungstag der Anmeldung: **22.09.2010**
(21) Anmeldenummer: 10401026.9
(22) Anmeldetag: 08.03.2010
(51) Int. Cl.: B05C 17/01, A61M 5/315, B05C 17/005

(54) **Spritze**

(30) Priorität: 19.03.2009 DE 102009013968
(71) Anmelder: fischerwerke GmbH & Co. KG, 72178 Waldachtal (DE)
(72) Erfinder: Schwaab, Frank, 79350 Sexau (DE); Assadi, Amir, 79312 Emmendingen (DE)

(57) **Zusammenfassung**

Die Erfindung betrifft eine Zweikomponenten-Einmalspritze (1). Die Erfindung schlägt vor, Kolbenstangen (4) zweier Kolben (3) der Spritze (1) mit Verzahnungen (12) zu versehen, die mit Sperrgliedern (11) zusammenwirken, die von einem Auspresshebel (7) abstehen, der gelenkig über Filmscharniere (9, 10) mit Zylindern (5) der Spritze (1) verbunden ist. Durch eine wiederholte, hin- und hergehende Schwenkbewegung des Auspresshebels (7) werden die Kolben (3) schrittweise in einer Auspressrichtung in die Zylinder (5) verschoben.

## Beschreibung

Die Erfindung betrifft eine Spritze mit einem Zylinder und mit einem in dem Zylinder verschiebbaren Kolben, mit den Merkmalen des Oberbegriffs des Anspruchs 1.

Spritzen sind beispielsweise als Einmalspritzen aus Kunststoff bekannt. Ihre Kolben weisen eine Kolbenstange, meist mit einer Abschlussplatte an einem dem Kolben fernen Ende auf, an der der Kolben in den Zylinder hineinverschiebbar ist, um eine im Zylinder enthaltene Substanz auszupressen. Ist die Substanz zähflüssig, beispielsweise teigig oder pastös, und/oder ist ein Statikmischer auf die Spritze aufgesetzt, um mehrere Komponenten miteinander zu mischen, ist die erforderliche Auspresskraft hoch. Auch mit dem Querschnitt der Spritze steigt die Auspresskraft.

Aufgabe der Erfindung ist, eine Spritze der vorstehend erläuterten Art vorzuschlagen, die das Auspressen erleichtert.

Diese Aufgabe wird erfindungsgemäß durch die Merkmale des Anspruchs 1 gelöst. Die erfindungsgemäße Spritze weist einen Auspresshebel auf, der gelenkig mit ihrem Zylinder verbunden ist. Der Auspresshebel weist ein Sperrglied auf, das mit der Kolbenstange in Eingriff bringbar ist. Das Sperrglied wirkt in einer Auspressrichtung, es überträgt eine Kraft vom Auspresshebel in der Auspressrichtung auf die Kolbenstange. In entgegengesetzter Richtung gelangt das Sperrglied außer Eingriff von der Kolbenstange. Durch eine hin- und hergehende Schwenkbewegung des Auspresshebels lässt sich der Kolben über seine Kolbenstange schrittweise in der Auspressrichtung verschieben und presst eine im Zylinder enthaltene Substanz aus der Spritze aus.

Sperrglieder sind von richtungsgeschalteten Kupplungen, die vielfach als Freiläufe bezeichnet werden, bekannt. Im Unterschied zu einer richtungsgeschalteten Kupplung überträgt hier das Sperrglied eine translatorische Bewegung in einer Verschieberichtung, nämlich der Auspressrichtung, und lässt eine Relativbewegung zwischen dem Sperrglied und der Kolbenstange in entgegengesetzter Richtung zu, während in richtungsgeschalteten Kupplungen eine Drehbewegung in einer Drehrichtung übertragen wird, wogegen eine Drehung in umgekehrter Richtung möglich ist. Mit dem Auspresshebel ist durch ein großes Hebelverhältnis eine große Auspresskraft möglich. Das Hebelverhältnis ist das Verhältnis der Längen wirksamer Hebelarme, mit denen beispielsweise eine Hand am Auspresshebel einerseits und das Sperrglied an der Kolbenstange andererseits angreifen. Die Erfindung ermöglicht das Auspressen selbst sehr zäher Substanzen aus der Spritze mit vergleichsweise geringer Handkraft. Selbst durch einen Statikmischer lassen sich zähe Substanzen auspressen.

Abzuheben ist für den Auspresshebel nicht auf dessen Form, er muss keine Form aufweisen, die man unbefangen als "Hebel" bezeichnen würde, sondern anhand seiner Funktion, durch hin- und hergehendes Schwenken die Kolbenstange schrittweise in der Auspressrichtung zu verschieben.

Ebenso wie bei richtungsgeschalteten Kupplungen eine kraftschlüssige, also durch Reibung bewirkte Übertragung des Drehmoments möglich ist, ist auch erfindungsgemäß ein kraftschlüssiger An- oder Eingriff des Sperrglieds an der Kolbenstange möglich, also eine Kraftübertragung in der Auspressrichtung durch Reibung. Eine Ausgestaltung der Erfindung sieht eine formschlüssige Kraftübertragung beispielsweise über eine Verzahnung der Kolbenstange vor.

Zur Erzielung einer großen Auspresskraft sieht eine Ausgestaltung der Erfindung ein großes Hebelverhältnis des Auspresshebels vor. Die gelenkige Verbindung des Auspresshebels befindet sich näher am Sperrglied als am anderen Ende des Auspresshebels, der Hebelarm des Sperrglieds ist kurz im Verhältnis zur Gesamtlänge des Auspresshebels.

Eine Ausgestaltung der Erfindung sieht eine gelenkige Verbindung des Auspresshebels mit dem Zylinder der Spritze über einen Schwenkhebel vor. Diese Ausgestaltung weist zwei Schwenkverbindungen auf, die eine verbindet den Schwenkhebel mit dem Zylinder, die andere den Schwenkhebel mit dem Auspresshebel. Nicht ausgeschlossen sind auch mehr als zwei Schwenkverbindungen. Der Schwenkhebel ermöglicht nicht nur die hin-und hergehende Schwenkbewegung des Auspresshebels zum schrittweisen Verschieben des Kolbens in der Auspressrichtung, sondern zusätzlich eine vorzugsweise kurze Bewegung quer zur Kolbenstange, durch die das Sperrglied bei Bewegung in der Auspressrichtung in Eingriff mit der Kolbenstange gelangt und bei entgegengesetzter Bewegung von der Kolbenstange freikommt.

Eine bevorzugte Ausgestaltung der Erfindung sieht eine einstückige Ausbildung des Zylinders mit dem Auspresshebel, vorzugsweise einschließlich des Sperrglieds, und, sofern vorhanden, einschließlich des Schwenkhebels, der den Auspresshebel gelenkig mit dem Zylinder verbindet, vor. Die einstückige Herstellung ist beispielsweise durch Spritzgießen aus Kunststoff möglich. Eine zweiteilige Ausbildung der Spritze ist dadurch möglich, nämlich der Zylinder mit dem Auspresshebel als ein Teil und der Kolben als zweites Teil.

Zu einer einfachen Handhabung sieht eine Ausgestaltung der Erfindung einen Handgriff am Zylinder vor, der gemeinsam mit dem Auspresshebel mit einer Hand umgriffen werden kann. Durch Schließen der Hand wird der Auspresshebel zum Handgriff hin verschwenkt und verschiebt die Kolbenstange mit dem Kolben in der Auspressrichtung. Beim Öffnen der Hand schwenkt der Auspresshebel zurück in die Ausgangsstellung, ohne den Kolben zurückzubewegen. Bei der Herstellung aus Kunststoff genügt die Elastizität des Kunststoffs, um den Auspresshebel beim Öffnen der Hand in die Ausgangsstellung zurückzuschwenken. Der Handgriff kann ebenfalls einstückiger Bestandteil des Zylinders sein. Er kann beispielsweise in Verlängerung des Zylinders nach hinten, d.h. entgegen der Auspressrichtung abstehen. Diese Ausgestaltung der Erfindung ist vergleichbar einer Schere mit einem feststehenden und einem beweglichen, nämlich schwenkbaren Scherenhebel.

Eine andere Ausgestaltung der Erfindung, die ebenfalls einer einfachen Handhabung der Spritze dient, sieht zwei gelenkig mit dem Zylinder verbundene Auspresshebel vor, die einander gegenüber angeordnet sind. Die Kolbenstange befindet sich zwischen den beiden Auspresshebeln, deren Sperrglieder von beiden Seiten an der Kolbenstange angreifen. Diese Ausgestaltung der Erfindung ist vergleichbar einer Schere mit zwei beweglichen, nämlich schwenkbar miteinander verbundenen Scherenhebeln. Sie hat den Vorteil eines symmetrischen Kraftangriffs der Sperrglieder an der Kolbenstange und einer Verdoppelung der Auspresskraft bei gegebener Handkraft.

Die Erfindung ist auch für Mehrkomponenten-Spritzen, beispielsweise Zweikomponenten-Spritzen mit einer entsprechenden Anzahl Zylindern und Kolben verwendbar. Es ist vorzugsweise ein gemeinsamer Auspresshebel oder auch zwei einander gegenüber angeordnete Auspresshebel vorhanden und es kann jeder Kolben eine Kolbenstange, die mit einer gleichen Anzahl Sperrglieder zusammenwirken, aufweisen. Die schrittweise Verschiebung der Kolben ist allerdings auch mit einer gemeinsamen Kolbenstange für alle Kolben möglich. Vorzugsweise sind die Kolben starr verbunden, wobei auch bei nicht verbundenen Kolben eine gleich große Verschiebung mit dem erfindungsgemäßen Auspresshebel möglich ist.

Die Erfindung wird nachfolgend anhand eines in der Zeichnung dargestellten Ausführungsbeispiels näher erläutert. Es zeigen:

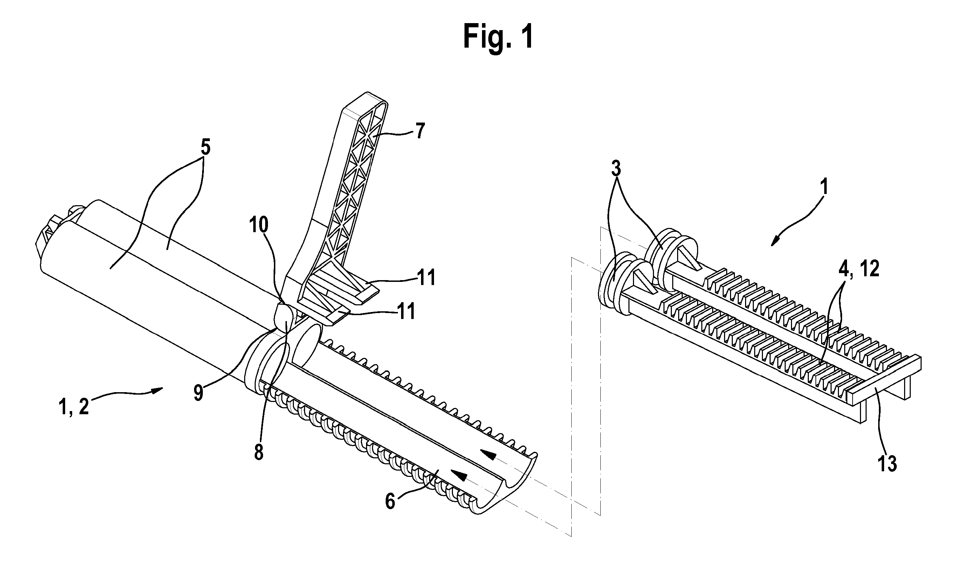

- Figur 1: eine Einzelteildarstellung einer Spritze gemäß der Erfindung in perspektivischer Darstellung;
- Figur 2: die Spritze aus Figur 1 in zusammengesetztem Zustand; und
- Figur 3: die Spritze aus Figur 2 mit anderer Blickrichtung.

Die in der Zeichnung dargestellte, erfindungsgemäße Spritze 1 ist eine Einmalspritze, d.h. sie ist zu einem einmaligen Gebrauch vorgesehen, und sie ist durch Spritzgießen aus Kunststoff hergestellt. Die Spritze 1 ist eine Zweikomponenten-Spritze, sie weist einen Doppelzylinder 2 und zwei Kolben 3 mit jeweils einer Kolbenstange 4 auf. Der Doppelzylinder 2 weist zwei Zylinder 5 auf, die parallel nebeneinander angeordnet und einstückig miteinander sind. Im Ausführungsbeispiel weisen die Zylinder 5 gleiche Durchmesser auf, es sind allerdings ebenso Zylinder mit verschiedenen Durchmessern möglich.

An einem hinteren, offenen Ende weist der Doppelzylinder 2 einen Handgriff 6 auf, der einstückig und starr mit den Zylindern 5 ist. Der Handgriff 6 hat die Form zweier nebeneinander liegender Halbzylinder in koaxialer Verlängerung der Zylinder 5 und mit gleichem Innendurchmesser wie diese. Diese Form ist nicht zwingend für den Handgriff 6.

Auf einer dem Handgriff 6 gegenüberliegenden Seite steht ein Auspresshebel 7 quer oder schräg von den Zylindern 5 ab. Der Auspresshebel 7 ist über einen Schwenkhebel 8 gelenkig mit dem hinteren, offenen Ende der beiden Zylinder 5 verbunden. Der Schwenkhebel 8 ist im Ausführungsbeispiel plattenförmig, er ist an einem Ende über ein Filmscharnier 9 wie bereits erwähnt schwenkbar mit dem hinteren Ende der beiden Zylinder 5 und mit einem weiteren Filmscharnier 10 an seinem anderen Ende schwenkbar mit dem Auspresshebel 7 verbunden. Die Filmscharniere 9, 10 weisen zueinander parallele Schwenkachsen auf, die Schwenkachse des Filmscharniers 9, das den Schwenkhebel 8 mit dem hinteren Ende der Zylinder 5 verbindet, verläuft tangential zu den beiden Zylindern 5. Die Filmscharniere 9, 10 sind durch Nuten dünne Materialstellen, die aufgrund der dünnen Materialausbildung das Schwenken ermöglichen. Der Auspresshebel 7 und der Schwenkhebel 8 sind mit dem Doppelzylinder 2 einstückig und in einem Arbeitsgang mit dem Doppelzylinder 2 durch Spritzgießen aus Kunststoff hergestellt.

Am Filmscharnier 10 ist der Auspresshebel 7 abgewinkelt, er weist dort zwei rechtwinklig von ihm abstehende Sperrglieder 11 auf. Im Ausführungsbeispiel sind die Sperrglieder 11 plattenförmig und ihre freien Enden weisen die Form von Zähnen wie bei einem Zahnrad auf. Die Sperrglieder 11 sind in einer gemeinsamen Ebene angeordnet, ihr Abstand voneinander entspricht einem Abstand der beiden Zylinder 5.

Die Kolbenstangen 4 weisen einen T-förmigen Querschnitt auf, Querhäupter der T-förmigen Kolbenstangen 4 sind mit Verzahnungen 12 nach Art von Zahnstangen versehen. An den Kolben 3 fernen Enden sind die Kolbenstangen 4 durch eine Traverse 13 verbunden.

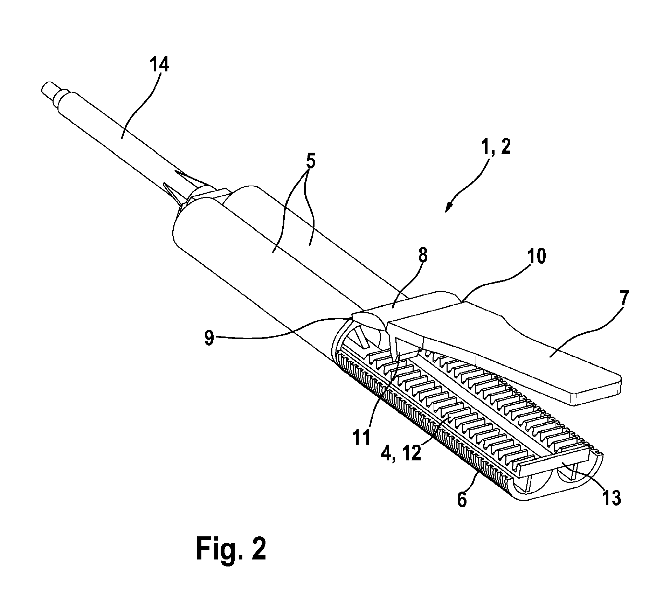

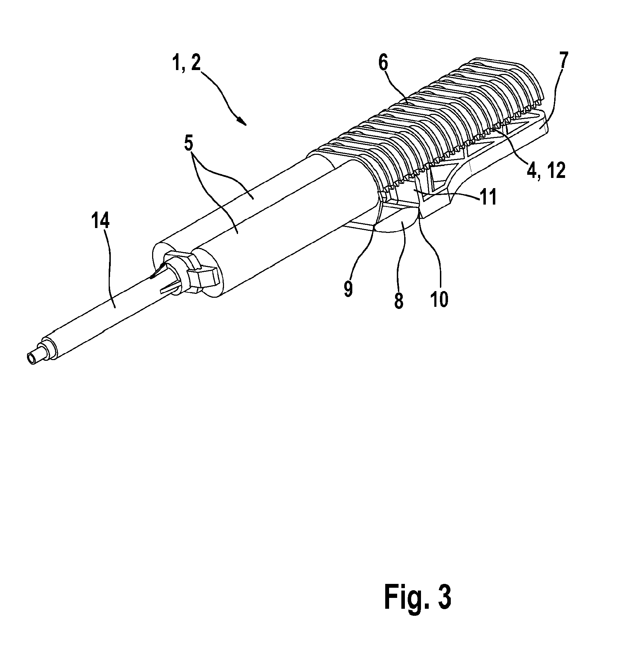

In zusammengesetztem Zustand befinden sich die Kolben 3 in den Zylindern 5 und die Kolbenstangen 4 liegen im Handgriff 6 ein, der den Kolbenstangen 4 eine Führung gibt und sie in einer Querrichtung abstützt. Die Verzahnungen 12 der Kolbenstangen 4 sind dem Handgriff 6 abgewandt. In Figuren 2 und 3 ist die Spritze 1 mit einem aufgesetzten Statikmischer 14 dargestellt. Solche Statikmischer sind an sich bekannt und sollen hier nicht erläutert werden, weil sie nicht Gegenstand der Erfindung sind. Zum Auspressen werden der Auspresshebel 7 und der Schwenkhebel 8 in Richtung des Handgriffs 6 geschwenkt, so dass die von dem Auspresshebel 7 abstehenden Sperrglieder 11 in Eingriff mit den Verzahnungen 12 der Kolbenstangen 4 gelangen, wie es in Figuren 2 und 3 zu sehen ist. Es werden der Auspresshebel 7 und der Handgriff 6 mit einer Hand umgriffen und zusammengedrückt. Dabei schwenkt der Auspresshebel 7 um das Filmscharnier 10, das ihn mit dem Schwenkhebel 8 verbindet. Dadurch schwenken die Sperrglieder 11 in Richtung der Zylinder 5 und verschieben durch ihren Eingriff in den Verzahnungen 12 die Kolbenstangen 4 mit den Kolben 3 in einer Auspressrichtung in die Zylinder 5 hinein. In den Zylindern 5 enthaltene Substanzen werden aus der Spritze 1 in den Statikmischer 14 gepresst. Beide Kolben 3 bewegen sich gleich weit, so dass die in den Zylindern 5 enthaltenen Substanzen immer im gleichen Verhältnis ausgepresst werden. Weisen die beiden Zylinder 5 gleiche Durchmesser auf, werden gleiche Mengen der beiden Substanzen ausgepresst.

Die nicht dargestellte, den Auspresshebel 7 und den Handgriff 6 umgreifende Hand wird geöffnet, wodurch aufgrund der Elastizität des Kunststoffs der Auspresshebel 7 vom Handgriff 6 weg verschwenkt. Auch der Schwenkhebel 8 schwenkt aufgrund der Elastizität des Kunststoffs so weit vom Handgriff 6 weg, dass die Sperrglieder 11 außer Eingriff von den Verzahnungen 12 der Kolbenstangen 4 gelangen. Durch erneutes Zusammendrücken des Auspresshebels 7 und des Handgriffs 6 gelangen die Sperrglieder 11 wieder in Eingriff mit den Verzahnungen 12, allerdings um einen oder mehrere Zähne weiter von den Kolben 3 entfernt als zuvor, weil die Kolben 3 und die Kolbenstange 4 beim vorausgegangenen Zusammendrücken des Auspresshebels 7 und des Handgriffs 6 wie beschrieben in der Auspressrichtung in die Zylinder 5 hinein verschoben worden sind. Beim erneuten Zusammendrücken des Auspresshebels 7 und des Handgriffs 6 verschiebt der Auspresshebel 7 über seine in die Verzahnungen 12 der Kolbenstangen 4 eingreifenden Sperrglieder 11 die Kolben 3 wieder ein Stück tiefer in die Zylinder 5 hinein. Durch wiederholtes Zusammendrücken und Lösen des Auspresshebels 7 und des Handgriffs 6 werden die beiden Kolben 3 schrittweise in die Zylinder 5 verschoben. Beim Zusammendrücken kommen die Sperrglieder 11 wie beschrieben in Eingriff mit den Verzahnungen 12, beim Loslassen schwenkt der Schwenkhebel 8 aufgrund der Elastizität des Filmscharniers 9 so weit vom Handgriff 6 weg nach außen, dass die Sperrglieder 11 von den Verzahnungen 12 der Kolbenstangen 4 freikommen und sich von den Zylindern 5 entfernen, ohne die Kolbenstangen 4 zu verschieben.

Die erfindungsgemäße Spritze ist auch mit einem Zylinder und einem Kolben als Einkomponenten-Spritze oder auch für mehr als zwei Komponenten ausführbar (nicht dargestellt). Anstatt des starr die Zylinder 5 nach hinten verlängernden Handgriffs 6 kann bei Ausführungsformen der Erfindung auch ein zweiter Auspresshebel vorgesehen sein, der wie der dargestellte Auspresshebel 7 über einen Schwenkhebel gelenkig mit den hinteren Enden der Zylinder 5 verbunden ist und der spiegelbildlich in entgegengesetzter Richtung von der Spritze 1 absteht wie der dargestellte Auspresshebel 7 (nicht dargestellt). Die Kolbenstangen 4 weisen in diesem Fall beidseitig Verzahnungen auf, die mit den Sperrgliedern der beiden einander gegenüber angeordneten Auspresshebel zusammenwirken. Handhabung und Funktion einer solchen, nicht dargestellten Spritze ist gleich wie beschrieben, es werden die beiden einander gegenüber angeordneten Auspresshebel zusammengedrückt, so dass ihre Sperrglieder mit den Verzahnungen der Kolbenstangen in Eingriff gelangen und die Kolben schrittweise in die Zylinder verschieben. Die Kolbenstangen befinden sich zwischen den beiden einander gegenüber angeordneten Auspresshebeln.

## Patentansprüche

1. Spritze mit einem Zylinder (5) und mit einem Kolben (3), der eine Kolbenstange (4) aufweist und der in dem Zylinder (5) verschiebbar ist, **dadurch gekennzeichnet, dass** die Spritze (1) einen Auspresshebel (7) aufweist, der gelenkig mit dem Zylinder (5) verbunden ist und der ein Sperrglied (11) aufweist, das in einer Auspressrichtung mit der Kolbenstange (4) in Eingriff bringbar ist, so dass durch eine hin- und hergehende Schwenkbewegung des Auspresshebels (7) der Kolben (3) schrittweise in der Auspressrichtung im Zylinder (5) der Spritze (1) verschiebbar ist.

2. Spritze nach Anspruch 1, **dadurch gekennzeichnet, dass** das Sperrglied (11) formschlüssig an der Kolbenstange (4) angreift.

3. Spritze nach Anspruch 2, **dadurch gekennzeichnet, dass** die Kolbenstange (4) eine Verzahnung (12) für den Eingriff des Sperrglieds (11) aufweist.

4. Spritze nach Anspruch 1, **dadurch gekennzeichnet, dass** der Auspresshebel (7) über einen Schwenkhebel (8) gelenkig mit dem Zylinder (5) verbunden ist.

5. Spritze nach Anspruch 1, **dadurch gekennzeichnet, dass** sich die gelenkige Verbindung (10) des Auspresshebels (7) mit dem Zylinder (5) der Spritze (1) näher am Sperrglied (11) als an einem dem Sperrglied (11) fernen Ende des Auspresshebels (7) befindet.

6. Spritze nach Anspruch 1, **dadurch gekennzeichnet, dass** der Auspresshebel (7) einstückig mit dem Zylinder (5) ist.

7. Spritze nach Anspruch 1, **dadurch gekennzeichnet, dass** der Zylinder (7) einen Handgriff (6) aufweist, der gemeinsam mit dem Auspresshebel (7) mit einer Hand umgriffen werden kann, so dass durch Schließen der Hand der Auspresshebel (7) in Richtung des Handgriffs (6) verschwenkbar ist, wobei das Sperrglied (11) in Eingriff mit der Kolbenstange (4) gelangt und diese mit dem Kolben (3) in der Auspressrichtung im Zylinder (5) verschiebt.

8. Spritze nach Anspruch 1, **dadurch gekennzeichnet, dass** die Spritze (1) zwei einander gegenüber angeordnete Auspresshebel (7) aufweist, zwischen denen die Kolbenstange (4) angeordnet ist.

9. Spritze nach Anspruch 1, **dadurch gekennzeichnet, dass** die Spritze (1) mehrere Zylinder (5) und eine gleiche Anzahl Kolben (3) aufweist, die gemeinsam mit dem Auspresshebel (7) verschiebbar sind.
